# EUROPEAN PATENT APPLICATION

(11) **EP 3 647 429 A1**
(43) Date of publication of application: **06.05.2020**
(21) Application number: 18203845.5
(22) Date of filing: 31.10.2018
(51) Int. Cl.: C12P 7/22

(54) **METHOD FOR THE ENZYMATIC PRODUCTION OF PHENOLIC COMPOUNDS**

(71) Applicant: Clariant International Ltd, 4132 Muttenz (CH)
(72) Inventor: TER HALLE, Robert, 81925 München (DE); LOEW, Sebastian, 81925 München (DE)
(74) Representative: Kampen, Daniela

(57) **Abstract**

The present invention refers to a method for the biocatalytic production of a phenolic compound such as resorcinol or a derivative thereof incubating a phenolic starting compound such as umbellic acid or umbelliferone with a cinnamate decarboxylase and a vinyl phenol reductase.

## Description

The present invention refers to a method for the biocatalytic production of a phenolic compound such as resorcinol or a derivative thereof incubating a phenolic starting compound such as umbellic acid or umbelliferone with a cinnamate decarboxylase and a vinyl phenol reductase.

### Technical background

Biocatalytic potential of microorganisms have been employed for centuries to produce bread, wine, vinegar and other common products without understanding the biochemical basis of their ingredients. Microbial enzymes have gained interest for their widespread uses in industries and medicine owing to their stability, catalytic activity, and ease of production and optimization compared to enzymes from plant or animal. The use of enzymes in various industries (e.g., food, agriculture, chemicals, and pharmaceuticals) is increasing rapidly due to reduced processing time, low energy input, cost effectiveness, nontoxic and eco-friendly characteristics. In recent years, the most significant development in the field of synthetic organic chemistry has been the application of biological systems to chemical reactions. Reactions catalyzed by enzymes and enzyme systems display far greater specificities than more conventional organic reactions. Biological and/or enzymatic syntheses and transformations, that is, "biotransformations," have great potential. Some of these reactions have already been shown to have useful applications in the fields of synthetic organic chemistry and biotechnology.

An example of chemical transformation is the production of resorcinol (benzene-1,3-diol) which is produced when any of a large number of resins (e.g., galbanum, asafoetida, etc.) are melted with potassium hydroxide, or by the distillation of Brazilwood extract. Alternatively, it is prepared synthetically by melting 3-iodophenol, phenol-3-sulfonic acid, or benzene-1,3-disulfonic acid with potassium carbonate; by the action of nitrous acid on 3-aminophenol or on 1,3-diaminobenzene. Many *ortho-* and *para*-compounds of the aromatic series (for example, the bromophenols, benzene-para-disulfonic acid) also yield resorcinol by reaction with potassium hydroxide.

Resorcinol is for example used as a chemical intermediate for the synthesis of pharmaceuticals and other organic compounds. It is used in the production of diazo dyes and plasticizers and as a UV absorber in resins. An emerging use of resorcinol is as a template molecule in supramolecular chemistry. The -OH groups on resorcinol form hydrogen bonds to target molecules, holding them in the proper orientation for a reaction. Many such reactions are able to be carried out in the solid state, thereby reducing or eliminating the use of solvents that may be harmful to the environment. Resorcinol is an analytical reagent for the qualitative determination of ketoses (e.g., Seliwanoff's test). It is the starting material for resorcinarene molecules and the initiating explosive lead styphnate. Further, it reacts with formaldehyde to form a thermoset resin which can form the basis of an aerogel.

Moreover, resorcinol is used in the cosmetic and medical field. Used externally, it is an antiseptic and disinfectant, and is used 5 to 10% in ointments in the treatment of chronic skin diseases such as psoriasis, hidradenitis suppurativa, and eczema of a sub-acute character. It is present in over-the-counter topical acne treatments at 2% or less concentration, and in prescription treatments at higher concentrations. Weak, watery solutions of resorcinol (25 to 35 g/kg) are useful in allaying the itching in erythematous eczema. It can be included as an anti-dandruff agent in shampoo or in sunscreen cosmetics. Resorcinol and some resorcinol derivatives, respectively, have tyrosine activity-inhibiting effect, which is used for example in a skin depigmental composition (US 4,959,393). In addition, they are used to treat hyperpigmentation disorders such as melasma, ephelide, freckles or solar lentigines (Garcia-Jimenez A. et al., Bioorganic & Medicinal Chemistry, 24, 2016, p. 4434-4443). In their effect on melatonin, resorcinol and its derivatives are used as whitener in cosmetic products.

So far, numerous phenolic compounds such as resorcinol and its derivatives are only synthetically produced for example for use in medical or cosmetic applications. Thus, there is a need for natural production of phenolic compounds such as resorcinol and its derivatives to avoid chemical side effects in the synthesis and to reach a very pure final product in a cost and time efficient method, which is even applicable in an industrial scale.

### Summary of the invention

The present invention refers to a method for biocatalytic production of a phenolic compound comprising the steps:
a) incubating a starting compound according to formula (Ia) 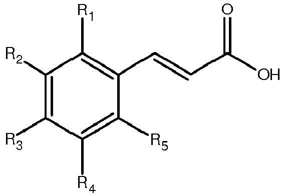 wherein at least one of R¹, R², R³, R⁴ or R⁵ is -OH and the other R¹, R², R³, R⁴ or R⁵ is -H, wherein optionally one or more of R¹, R², R³, R⁴ or R⁵ is -OCH₃ instead of -H, or formula (Ib) 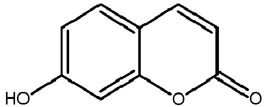 or a combination thereof with cinnamate decarboxylase, and
b) further incubating the starting compound and/or an intermediate thereof with vinyl phenol reductase.

The starting compound is for example umbellic acid, umbelliferone, o-coumaric acid, m-coumaric acid, p-coumaric acid, ferulic acid, sinapic acid, caffeic acid, 2,5-dihydroxycinnamic acid, or a combination thereof. The phenolic compound is for example 4-ethyl resorcinol, 4-ethylphenol, 4-ethylguaiacol, 4-ethylsyringol, 4-ethylcatechol, 2-ethylbenzene-1,4-diol, a derivative thereof, or a combination thereof.

The starting compound is for example incubated with a microorganism expressing cinnamate decarboxylase and/or vinyl phenol reductase, is incubated with an isolated cinnamate decarboxylase and/or vinyl phenol reductase, is incubated with a cell-free expressed cinnamate decarboxylase and/or vinyl phenol reductase, is incubated with a synthetically produced cinnamate decarboxylase and/or vinyl phenol reductase, or a combination thereof. The cinnamate decarboxylase, the vinyl phenol reductase or both are for example natively or recombinant expressed in the microorganism, which is for example a fungi such as yeast, or bacteria such as *Escherichia coli* or *Bacillus subtilis,* in particular in case of recombinant expression.

The cinnamate decarboxylase, the vinyl phenol reductase or both are for example a native form or a genetically modified form.

The yeast expressing cinnamate decarboxylase, vinyl phenol reductase or both is for example *Brettanomyces,* also called *Dekkera,* such as *Brettanomyces (Dekkera) bruxellenis* or *Brettanomyces (Dekkera) anomalus, Pichia* such as *Pichia guilliermondii,* or *Candida* such as *Candida versatilis, Candida halophile* or *Candida mannitofaciens,* or a combination thereof.

The cinnamate decarboxylase and the vinyl phenol reductase are for example either added to the starting compound together or separately for example at the same time or at different time points for example wherein vinyl phenol reductase is added, e.g., 1, 2, 5, 10, 12, 15 or 24 h after the cinnamate decarboxylase.

The temperature is for example maintained during step a), b) or both steps in a range from 0 to 100 °C, 5 to 99 °C, 10 to 95 °C, 15 to 90 °C, 20 to 85 °C, 20 to 60 °C, 25 to 50 °C, 30 to 40 °C, 30 to 80 °C, 35 to 75 °C, 40 to 65 °C or 45 to 60 °C or 0 °C, 5 °C, 10 °C, 15 °C, 20 °C, 25 °C, 30 °C, 35 °C, 40 °C, 45 °C, 50 °C, 55 °C, 60 °C, 65 °C, 70 °C, 75 °C, 80 °C, 85 °C, 90 °C, 93 °C, 95 °C, 98 °C, 99 °C or 100°C, and the given temperature is for example maintained for 1 h, 3 h, 5 h, 10 h, 15 h, 20 h, 24 h, 30 g, 36 h, 42 h, 48 h, 54 h or 60 h, 3 days, 4 days, 5 days, 6 days, 7 days, 8, days, 9 days or 10 days.

The method of the present invention is for example performed in an industrial scale.

All documents cited or referenced herein ("herein cited documents"), and all documents cited or referenced in herein cited documents, together with any manufacturer's instructions, descriptions, product specifications, and product sheets for any products mentioned herein or in any document incorporated by reference herein, are hereby incorporated herein by reference, and may be employed in the practice of the invention. More specifically, all referenced documents are incorporated by reference to the same extent as if each individual document was specifically and individually indicated to be incorporated by reference.

### Description of figures

**Fig. 1** shows a GC-MS analysis of the phenolic compound 4-ethyl resorcinol (4-ER) after different times of incubation of umbellic acid with *Brettanomyces (Dekkera) bruxellenis* natively expressing cinnamate decarboxylase and vinyl phenol reductase.
**Fig. 2** depicts a kinetic profile of the formation of 4-ethyl resorcinol over time.

### Detailed description

The present invention refers to a method for biocatalytic, e.g., enzymatic production of a phenolic compound such as 4-ethyl resorcinol, 4-ethylphenol, 4-ethylguaiacol, 4-ethylsyringol, 4-ethylcatechol, 2-ethylbenzene-1,4-diol, a derivative thereof, or a combination thereof. Enzymes used in this method are for example cinnamate decarboxylase and vinyl phenol reductase. Starting compounds being incubated with these enzymes are for example umbellic acid, umbelliferone, o-coumaric acid, m-coumaric acid, p-coumaric acid, ferulic acid, sinapic acid, caffeic acid, 2,5-dihydroxycinnamic acid or a combination thereof. For example incubation of umbellic acid, umbelliferone or both with cinnamate decarboxylase and vinyl phenol reductase results in the biocatalytic production of 4-ethyl resorcinol. The method of the present invention is applicable for industrial production of a phenolic compound.

In the following, the elements of the present invention will be described in more detail. These elements are listed with specific embodiments, however, it should be understood that they may be combined in any manner and in any number to create additional embodiments. The variously described examples and embodiments should not be construed to limit the present invention to only the explicitly described embodiments. This description should be understood to support and encompass embodiments which combine the explicitly described embodiments with any number of the disclosed elements. Furthermore, any permutations and combinations of all described elements in this application should be considered disclosed by the description of the present application unless the context indicates otherwise.

Throughout this specification and the claims, unless the context requires otherwise, the word "comprise", and variations such as "comprises" and "comprising", will be understood to imply the inclusion of a stated member, integer or step or group of members, integers or steps but not the exclusion of any other member, integer or step or group of members, integers or steps. The terms "a" and "an" and "the" and similar reference used in the context of describing the invention (especially in the context of the claims) are to be construed to cover both the singular and the plural, unless otherwise indicated herein or clearly contradicted by the context. Recitation of ranges of values herein is merely intended to serve as a shorthand method of referring individually to each separate value falling within the range. Unless otherwise indicated herein, each individual value is incorporated into the specification as if it were individually recited herein. All methods described herein can be performed in any suitable order unless otherwise indicated herein or otherwise clearly contradicted by context. The use of any and all examples, or exemplary language (e.g., "such as", "for example"), provided herein is intended merely to better illustrate the invention and does not pose a limitation on the scope of the invention otherwise claimed. No language in the specification should be construed as indicating any non-claimed element essential to the practice of the invention.

According to the method of the present invention, the starting compound is for example incubated with a microorganism expressing cinnamate decarboxylase and/or vinyl phenol reductase or is incubated with an isolated cinnamate decarboxylase and/or vinyl phenol reductase, is incubated with a cell-free expressed cinnamate decarboxylase and/or vinyl phenol reductase, or is incubated with a synthetically produced cinnamate decarboxylase and/or vinyl phenol reductase, or a combination thereof. For example a microorganism expressing cinnamate decarboxylase and/or vinyl phenol reductase is combined with an isolated cinnamate decarboxylase and/or vinyl phenol reductase, a cell-free expressed cinnamate decarboxylase and/or vinyl phenol reductase, or a synthetically produced cinnamate decarboxylase and/or vinyl phenol reductase or a combination thereof in a method of the present invention. Alternatively, an isolated cinnamate decarboxylase and/or vinyl phenol reductase is combined with a cell-free expressed cinnamate decarboxylase and/or vinyl phenol reductase or a synthetically produced cinnamate decarboxylase and/or vinyl phenol reductase or a combination thereof in a method of the present invention. Alternatively, a cell-free expressed cinnamate decarboxylase and/or vinyl phenol reductase is combined with a synthetically produced cinnamate decarboxylase and/or vinyl phenol reductase.

The cinnamate decarboxylase, the vinyl phenol reductase or both are for example natively or recombinant expressed in a microorganism, which is for example a fungi such as yeast, or bacteria, e.g., *Escherichia coli* or *Bacillus subtilis,* in particular in case of recombinant expression, or a combination thereof. The isolated cinnamate decarboxylase and/or vinyl phenol reductase is for example isolated from a natively or recombinant expressing microorganism, or both. Successful enzymatic reaction of a starting compound such as umbellic acid and/or umbelliferone into a phenolic compound such as 4-ethyl resorcinol takes for example place in a growing and/or a resting microorganism. The resting stadium of a microorganism is for example reached in a minimal medium, where a nutrition source such as nitrogen and/or phosphate is missing.

The cinnamate decarboxylase, the vinyl phenol reductase or both are for example a native form or a genetically modified form, e.g., resulting in an increased expression of the cinnamate decarboxylase, the vinyl phenol reductase or both. The efficacy and/or the specificity of the native and/or the recombinant enzyme is for example altered (e.g., increased) by directed evolution or any other type of mutagenesis know to a person skilled in the art or a combination thereof.

The yeast expressing cinnamate decarboxylase, vinyl phenol reductase or both is for example *Brettanomyces,* also called *Dekkera,* such as *Brettanomyces (Dekkera) bruxellenis* or *Brettanomyces (Dekkera) anomalus, Pichia* such as *Pichia guilliermondii,* or *Candida* such as *Candida versatilis, Candida halophile* or *Candida mannitofaciens,* or a combination thereof.

The cinnamate decarboxylase and the vinyl phenol reductase are for example either added to the starting compound together or separately for example in the same or different microorganisms natively or recombinant expressed or in combination with an isolated and/or synthetic cinnamate decarboxylase and/or vinyl phenol reductase.

The cinnamate decarboxylase and the vinyl phenol reductase are added to the starting compound for example at the same time or at different time points, for example wherein vinyl phenol reductase is added e.g., 1, 2, 5, 10, 12, 15 or 24 h after the cinnamate decarboxylase. The vinyl phenol reductase for example is added to the starting compound and/or an intermediate resulting from the incubation of the starting compound with cinnamate decarboxylase, after minutes, hours, days or weeks from addition of the cinnamate decarboxylase.

The temperature is for example maintained during step a), b) or both steps in a range from 0 to 100 °C, 5 to 99 °C, 10 to 95 °C, 15 to 90 °C, 20 to 85 °C, 20 to 60 °C, 25 to 50 °C, 30 to 40 °C, 30 to 80 °C, 35 to 75 °C, 40 to 65 °C or 45 to 60 °C, or 0 °C, 5 °C, 10 °C, 15 °C, 20 °C, 25 °C, 30 °C, 35 °C, 40 °C, 45 °C, 50 °C, 55 °C, 60 °C, 65 °C, 70 °C, 75 °C, 80 °C, 85 °C, 90 °C, 93 °C, 95 °C, 98 °C, 99 °C or 100°C, and the given temperature is for example maintained for 1 h, 3 h, 5 h, 10 h, 15 h, 20 h, 24 h, 30 g, 36 h, 42 h, 48 h, 54 h or 60 h, 3 days, 4 days, 5 days, 6 days, 7 days, 8, days, 9 days or 10 days. Alternatively, the temperature may vary over the process time for example between step a) and step b) of the method of the present invention.

The method of the present invention is for example performed in an industrial scale. Industrial production of a phenolic compound according to the method of the present invention for example takes place in a fermenter for example in a batch process, fed-batch process, continuous process or combinations thereof. The fermenter size is for example 0.1; 0.2; 0.3; 0.4; 0.5; 0.6; 0.7; 0.8; 0.9; 1, 3, 5, 10 20, 30, 40, 50, 60, 70, 80, 90, 100, 200, 300, 400, 500, 600, 700, 800, 900 or 1000 m³.

### Examples

The following examples illustrate different embodiments of the present invention, but the invention is not limited to these examples.

### Example 1: Brettanomyces biomass production

The strain CBS4481 of *Brettanomyces* was recovered in YPG-medium (yeast, peptone, glucose) and plated on YPG-agar. One colony was picked after one week and cultivated in 20 ml YPG-medium for growing the colony. Samples of this colony were used in experiments for the following examples and were stored in glycerol stocks, respectively.

### Example 2: Bioconversion experiments using growing Brettanomyces under standard conditions

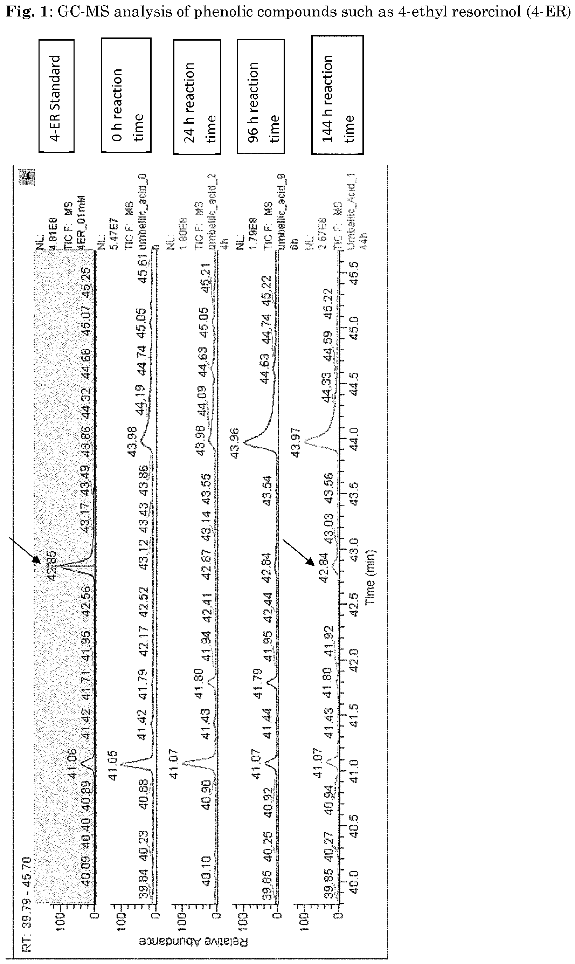

50 ml YPG medium in an Erlenmeyer flask was inoculated with 500 µl from the initial culture of Brettanomyces described in Example 1 1 ml of this pre-culture was used to inoculate an Erlenmeyer flask containing 50 ml of YPG medium. To the flask 100 µl ethanol/water (70:30 *v*/*v*) containing substrate e.g. coumaric acid or umbellic acid was added for a final substrate concentration of 1 mM. The cultures were incubated at 30 °C for 5 days. 500 µl of the samples were taken regularly and added to 500 µl of ethyl acetate. After mixing vigorously, 100 µl of the organic layer was transferred to a glass vial and analyzed by GC-MS (see Fig. 1).

### Example 3: Bioconversion experiments using Brettanomyces under modified conditions

50 ml YPG medium in an Erlenmeyer flask was inoculated with 500 µl from the initial culture of *Brettanomyces* described in Example 1. 10 ml of this pre-culture was used to inoculate an Erlenmeyer flasks containing 500 ml of YPG medium. The cells were harvested *via* centrifugation and washed three times with sodium phosphate buffer (50 mM, pH 7.5).

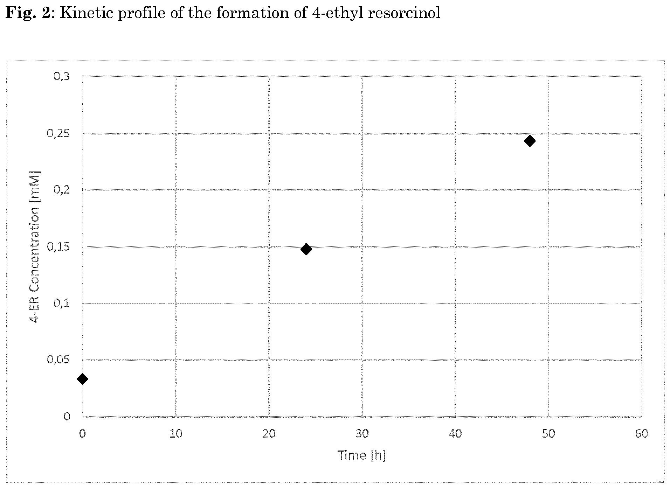

The obtained cells were then suspended at 1 g/l in sodium phosphate buffer (50 mM, pH 7.5) containing 20 g/l of glucose, 5 vol% of DMSO and 10 mM of substrate, i.e., coumaric acid or umbellic acid. The reaction was incubated in a closed falcon tube at 30 °C and mild shaking for two days. At regularly intervals the samples were analyzed by GC-MS as described in Example 2 (see Fig. 2).

## Claims

1. Method for biocatalytic production of a phenolic compound comprising the steps:
a) incubating a starting compound according to formula (Ia) 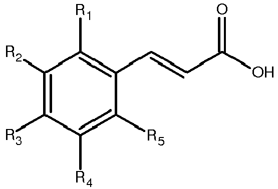 wherein at least one of R¹, R², R³, R⁴ or R⁵ is -OH and the other R¹, R², R³, R⁴ or R⁵ is -H, wherein optionally one or more of R¹, R², R³, R⁴ or R⁵ is -OCH₃ instead of -H, or formula (Ib) 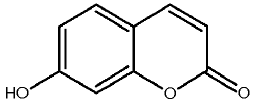 or a combination thereof, with cinnamate decarboxylase, and
b) further incubating the starting compound and/or an intermediate thereof with vinyl phenol reductase.

2. Method according to claim 1, wherein the starting compound is 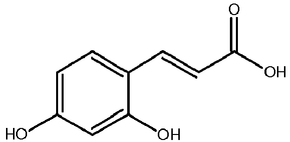 umbellic acid, 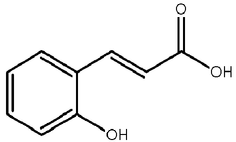 o-coumaric acid, 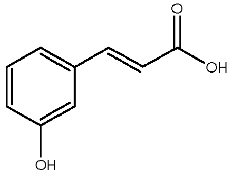 m-coumaric acid, 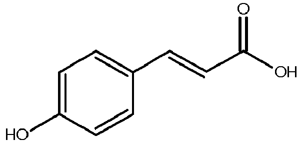 p-coumaric acid, 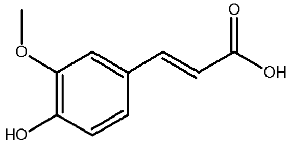 ferulic acid, 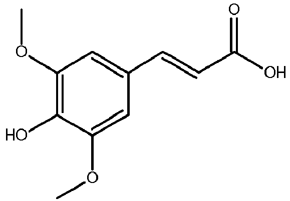 sinapic acid, 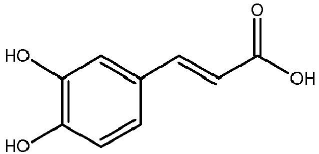 caffeic acid, 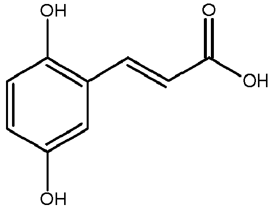 2,5-dihydroxycinnamic acid or a combination thereof.

3. Method according to claim 1 or 2, wherein the phenolic compound is 4-ethyl resorcinol, 4-ethylphenol, 4-ethylguaiacol, 4-ethylsyringol, 4-ethylcatechol, 2-ethylbenzene-1,4-diol, a derivative thereof, or a combination thereof.

4. Method according to any one of claims 1 to 3, wherein the starting compound is incubated with a microorganism expressing cinnamate decarboxylase and/or vinyl phenol reductase, is incubated with an isolated cinnamate decarboxylase and/or vinyl phenol reductase, is incubated with a cell-free cinnamate decarboxylase and/or vinyl phenol reductase, is incubated with a synthetically produced cinnamate decarboxylase and/or vinyl phenol reductase, or a combination thereof.

5. Method according to any one of claims 1 to 4, wherein the cinnamate decarboxylase, the vinyl phenol reductase or both are natively or recombinant expressed.

6. Method according to any one of claims 1 to 5, wherein the cinnamate decarboxylase, the vinyl phenol reductase or both are a native form or a genetically modified form.

7. Method according to any one of claims 1 to 6, wherein the microorganism expressing the cinnamate decarboxylase, the vinyl phenol reductase or both is a yeast.

8. Method according to claim 7, wherein the yeast is *Brettanomyces (Dekkera)* such as *Brettanomyces (Dekkera) bruxellenis* or *Brettanomyces (Dekkera) anomalus, Pichia* such as *Pichia guilliermondii,* or *Candida* such as *Candida versatilis, Candida halophile* or *Candida mannitofaciens,* or a combination thereof.

9. Method according to any one of claims 1 to 8, wherein the cinnamate decarboxylase and the vinyl phenol reductase are added to the starting compound together or separately.

10. Method according to claim 9, wherein the cinnamate decarboxylase and the vinyl phenol reductase are added at the same time or at different time points.

11. Method according to any one of claims 1 to 10, wherein the temperature is maintained during step a), b) or both steps in a range from 0 to 100 °C, 5 to 99 °C, 10 to 95 °C, 15 to 90 °C, 20 to 85 °C, 20 to 60 °C, 25 to 50 °C, 30 to 40 °C, 30 to 80 °C, 35 to 75 °C, 40 to 65 °C or 45 to 60 °C.

12. Method according to claim 11, wherein the given temperature is maintained for 1 h, 3 h, 5 h, 10 h, 15 h, 20 h, 24 h, 30 h, 36 h, 42 h, 48 h, 54 h, 60 h, 3 days, 4 days, 5 days, 6 days, 7 days, 8, days, 9 days or 10 days.
